# EUROPEAN PATENT APPLICATION

(11) **EP 2 000 159 A1**
(43) Date of publication of application: **10.12.2008**
(21) Application number: 07425359.2
(22) Date of filing: 07.06.2007
(51) Int. Cl.: A61M 1/10

(54) **A duct for a ventricular assistance device**

(71) Applicant: NewCorTec S.p.A., 06034 Foligno (PG) (IT)
(72) Inventor: Ercolani, Mauro, 00143 Roma (IT); Minoletti, Francesco, 10149 Torino (IT); Torriani, Flavio, 00132 Roma (IT); Rinaldi, Stefano, 43100 Parma (IT)
(74) Representative: Bosotti, Luciano

(57) **Abstract**

A duct (10) for carrying blood for ventricular-assistance devices (VADs) has a tubular body (28) and an end (12) having associated thereto a valve (18) for enabling the flow of blood unidirectionally in the duct (10). The valve (18) has an annular reinforcement (20), and the end (12) of the duct (10) is provided with a slot (22a, 24a) for receiving the annular reinforcement (20) of the valve (18) with the reinforcement in a retracted position in said slot (22a, 24a). The aforesaid end (12) and the aforesaid tubular body (28) of the duct are provided with a continuous coating (38, 40) of haemocompatible material, such as a polyurethane.

## Description

### Field of the invention

The invention relates to cardiac ventricular-assistance devices, commonly referred to as VADs.

### Description of the related art

Cardiac ventricular-assistance devices are well known in the art and are used for replacing or helping one of the ventricles of the heart, usually the left one, in its function of pumping the blood, thus reducing the work thereof. VADs are then pumps for the blood.

Different types of VADs are known in the art. In particular, the action of the VAD can generate a pulsatile flow of blood similar to that of the natural heart (pulsatile VADs) or substantially continuous (continuous-flow VADs). In the first case, the pump of the VAD, which takes the blood from a ventricle of the heart and pushes it into the peripheral or pulmonary circulation, is formed by a deformable chamber, provided with inlet and outlet ducts and one-way valves, which is cyclically compressed and expanded; in the latter case, the pump is of a centrifugal or axial type (turbine).

The pumping element of the VAD can moreover be implanted in the body of the patient or kept on the outside and connected to the cardiocirculatory system by means of ducts that traverse the skin; in this connection, the terms of "implantable VADs" and "paracorporeal VADs" are respectively used.

The present invention relates to VADs of a pulsatile type, which are preferentially, but not exclusively, implantable, in particular in the version comprising a pumping element with pulsatile sac. Devices of this type are known, for example, from the documents Nos. EP-A-0 728 488, EP-A-0 728 489, EP-A-1 066 840, EP-A-1 466 635, and again by the European patent application No. 06425592.0.

These are, in all the cases illustrated in the documents cited above, devices with operation of a pulsed type, designed principally to pump the blood from the left ventricle to the aorta of the patient. For this purpose, the ventricular-assistance device comprises two ducts or cannulae, designed:
- the first, to carry the blood of the patient from one ventricle, usually the left one (usually starting from an opening formed in an apical position in the ventricle itself) to the pumping element of the VAD; and
- the second, to carry the blood from the pumping element of the VAD to an artery, usually the aorta.

To ensure proper development of the pumping action of the VAD, associated to the end of the cannulae coming under the implantable pump are one-way valves. For this function, usually cardiac valve prostheses are used, both of a mechanical type (for example, with tilting disk) and of a biological type.

Various solutions for coupling between valves and cannulae or ducts (hereinafter the two terms will be used equivalently) are illustrated in documents, such as US-A-4 086 665, US-A-4 118 806, and again US-B-6 001 056.

The above solutions also form the subject of an extensive scientific literature. By way of example, it is possible to cite the following documents:
- R.D. Dowling et al., "HeartMate® VE LVAS design enhancements and its impact on device reliability", European Journal of Cardio-thoracic Surgery 25 (2004), pp. 958-963,
- T. Mussivand, et al.: "Clinical results with an ePTFE inflow .conduit ' for mechanical circulatory support" - The Journal of Heart and Lung Transplantation, Volume 23, Issue 12, December 2004, pp. 1366-1370.

Considering the method of implantation, the connection of the cannulae to the pumping element of the ventricular-assistance device is usually entrusted to the surgeon. It is thus necessary for the cannulae (and the valves associated thereto) to be connectable to the pumping element of the VAD in a relatively easy way, implementable in a safe and reliable way; this also as regards the need to prevent undesirable inclusion of air in the blood circuit.

Furthermore, it is desirable that the ventricular-assistance device, including the cannulae, should present excellent qualities of haemocompatibility.

In the case of ventricular-assistance devices for which it is envisaged that the pumping element will be implanted in the body of the patient, it is then important to ensure that the cannulae, which are to extend through the diaphragm of the patient between the thoracic cavity and the abdominal position, where the pumping element is usually implanted, are not subject to undesired phenomena of squeezing such as to hinder blood circulation.

For this reason, it is known to resort to supporting reinforcements (consisting basically of helical wires, which support the wall of the cannula at least locally). The latter consequently assumes a corrugated appearance, typically with a helical corrugation, substantially resembling the corrugated configuration traditionally adopted for so-called vascular grafts of textile material.

With reference to the vascular grafts, there is on the other hand known the solution of integrating a cardiac valve prosthesis directly in a vascular graft, for example so as to provide a composite implantation device that is usable, for example, for the surgical replacement of the aortic valve and of a stretch of the aorta, for example the ascending one or the entire aortic arch.

In the application to cardiac ventricular-assistance devices, it is, however, necessary to take into account the fact that the action of the pumping element of the device can be particularly energetic (it being obtained, for example, via a pumping element with electrical actuation), so that the end stretch of the cannula, which carries associated thereto the valve, may be exposed to mechanical stresses of a certain magnitude, particularly in the case of the duct that carries the blood from the heart to the pumping element.

### Object and summary of the invention

From the foregoing, there is felt the need to have available ducts with associated valves (in brief, "valved" ducts) that are able to meet in an excellent way all the needs - frequently contrasting with one another - outlined previously.

The object of the present invention is to meet that need.

According to the present invention, that object is achieved thanks to a valved duct (or cannula) having the characteristics recalled specifically in Claim 1. Advantageous developments of the invention form 'the subject of the claims depending thereon.

The claims form an integral part of the disclosure of the invention provided herein.

### Brief description of the annexed figures

The invention will now be described, purely by way of non-limiting example, with reference to the annexed figures, in which:
- Figure 1 is a schematic illustration of a VAD system complete with its implantable components (pumping element and cannulae) and with the ones that can be worn necessary for supply and control of the pump;
- Figure 2 illustrates more clearly the ensemble of the implantable parts;
- Figure 3, comprising two parts designated by a) and b), shows an example of the inlet and outlet ducts of a VAD;
- Figure 4 is an axial cross section of the end of a duct for a cardiac ventricular-assistance device that is to be connected to the pumping element of a VAD; and
- Figure 5 presents the scheme of the apparatus that can be used for coating with haemocompatible material the internal surface of ducts, such as the ones illustrated in the previous figures.

### Detailed description of exemplary embodiments

In the figures of the annexed drawings, the reference number 10 designates as a whole a duct or cannula that can be used in association with a ventricular-assistance device (VAD) of the pulsatile type, such as the ones described in any of the European patent documents originally cited in the introductory part of the present description. The duct 10 can then be any one of the ducts coming under the pumping element of the device.

In particular, the duct 10 has:
- a first end 12, which is to be connected to the pumping element of the device; and
- a second end 14, which is to be connected to the left ventricle of the patient (via a suture ring clearly visible in the drawings) or else to the aorta of the patient himself, according to the function of the duct 10.

The solution described herein relates specifically to the first end, designated by 12. It follows that the solution described herein may be applied indifferently either to the cannula that carries the blood of the patient (coming from the left ventricle) to the device or to the cannula that carries the blood from the ventricular-assistance device to the aorta of the patient. More in general, the solution described herein is suited to being used also in the case of ducts/cannulae that are to be implanted in conditions different from the ones described previously purely by way of example.

In this regard, persons skilled in the sector will understand that the characteristics of the pumping element of the ventricular-assistance device do not have in itself any specific importance for the purposes of the solution described herein. It will thus be merely assumed that the end 12 of the duct 10 is to be connected to a connector carried by the casing of the pumping element of the device. In the example illustrated herein, said connector comprises an externally threaded tubular shank C so as to enable connection of the duct 10 through an internally threaded annular ring nut 16 so as to enable it to be screwed on the shank C.

Even though this is not at the moment a preferred solution, the arrangement of coupling elements described herein could be, at least partially, reversed - except for what is said specifically in what follows.

Preferentially, and for reasons that will emerge more clearly in what follows, it will likewise be assumed that the internal surface and also the end edge of the tubular connector C have a coating made of haemocompatible material C1. Said material is constituted typically by a haemocompatible polymer, such as a polyurethane. The aforesaid haemocompatible coating C1 can be advantageously constituted by an integral extension of the pumping sac (not illustrated in the drawings, but of a type in itself known) of the ventricular-assistance device.

Associated to the end 12 of the duct 10 is a valve 18 constituted typically by a cardiac valve prosthesis. The annexed drawing refers to a valve prosthesis of a mechanical type, for example of the type with tilting disk. Of course, a choice of this sort is by no means imperative.

It will likewise be appreciated that, without prejudice to the other characteristics, the valve 18 will be mounted with different orientations, opposite to one another, according to the function of the duct 10.

In the case of a duct 10, through which the blood of the patient flows to the pumping element of the device, the valve 18 will be oriented so as to enable flow of blood from the end 14 to the end 12 and to prevent flow in the opposite direction.

In the case of a duct 10 that is to carry the blood from the pumping element of the device to the vascular system of the patient, the valve 18 will instead be oriented in such a way as to enable flow from the end 12 to the end 14 and to prevent flow in the opposite direction.

Since it is a cardiac valve prosthesis, the valve 18 will usually have an annular reinforcement 20 with an outer peripheral groove 20a normally designed to house the suture ring of the valve; said suture ring has no reason to be present in the case of the condition of use illustrated herein.

The reference numbers 22 and 24 designate two, as a whole annular, bodies designed to constitute the basic structure of the end 12 of the duct. These are preferably shaped bodies made of plastic material (for example, a thermoplastic polyurethane, such as Tecoplast or else polyethylene terephthalate or PTFE), provided with an external coating of haemocompatible material, constituted typically by a haemocompatible polymer, such as a polyurethane.

The bodies 22 and 24 can be connected to one another in a relationship of grafting and have mutually facing grooved parts 22a, 24a, designed to perform a relationship of gripping and of shape fit with the reinforcement 20 of the prostheses 18. The grooves 22a and 24a hence jointly define an annular slot designed to receive within it the reinforcement 20 of the valve 18, at the same time causing the reinforcement 20 to be in a retracted position in the aforesaid slot, i.e., the rim of the internal orifice of the valve of which, as a whole, projects slightly (in an internal radial direction) and, in general, radiused without formations of discontinuity of section with the adjacent areas of the elements 22 and 24.

The reference 26 designates a further annular body, which has a central orifice 26a that is generally flared according to a pattern diverging towards the bodies 22 and 24 (and hence towards the valve 18).

The reference 28 designates the body of the duct 10, constituted, for example, by a tubular element made of plastic material, such as for example foamed polytetrafluoroethylene (ePTFE, Goretex™). Within it, the body 28 as a whole has a smooth surface except for the corrugations given by the presence of an outer reinforcement structure 30 (for example, a helical metal-wire structure) designed to protect the body 28 of the duct from undesirable phenomena of squeezing, for example in the stretch that, in the position of implantation, is designed to extend in the proximity of the thoracic cage of the patient.

The end of the body 28, which is to face the valve 18, is also slightly flared, hence divaricated, as a result of the connection (usually obtained adhesively) with the diverging internal wall of the central orifice 26a of the element 26.

The end of the body 28 received within the element 26 (and, marginally, also the element 26 itself) undergo an operation of cutting designed to form a front contrast surface 28a, designed to rest precisely against the homologous edge of the body 24.

The ensemble constituted by the bodies 22 and 24 with the valve 18 and the ensemble of the element 26 with the body 28 are joined to one another precisely by means of a tubular element 29, usually glued on the outside of the elements 26 and 24.

A further tubular element 31 withholds the end of the outer reinforcement structure 30 in a purposely provided groove of the element 26.

The reference number 32 designates a ring that functions as spacer for carrying the ensemble constituted by the elements 22-20-24-26-31-32 to a precisely determined axial length. The spacer ring 32 is designed to co-operate in a relationship of thrust between the ensemble 22-20-24-26-31, on the one hand, and a tubular sleeve 34, on the other, fitted around the end 12 of the duct 10 and which has within it an annular flange 36. The flange 36 forms a contrast surface, designed to co-operate in a relationship of thrust against the spacer ring 32.

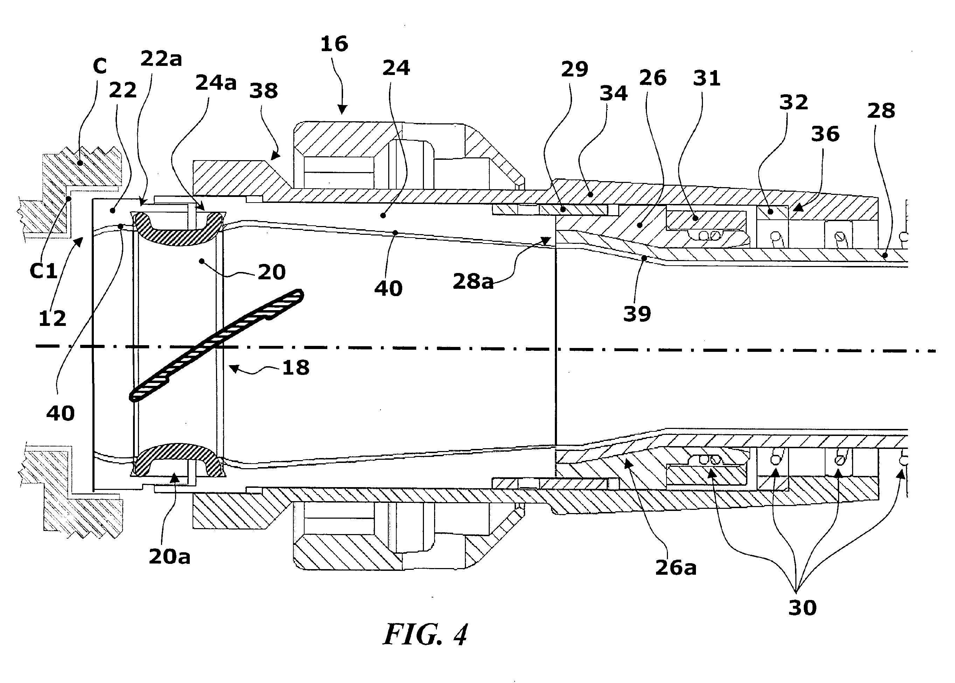

In this regard, it will be appreciated that - for reasons of clarity of illustration - in the cross section of Figure 4, the ring 32 has been represented at a certain distance from the elements 26 and 31 facing it. In use (and as will be further clarified in what follows), i.e., when the ring nut 16 is screwed on the shank C, the ring 32 is brought, instead, into a position of contrast against said elements, as schematically represented by a dashed line in the same Figure 4.

The tubular sleeve 34 has, on its outer surface, at the opposite end with respect to the flange 36, hence at the end facing the valve 18, an annular relief 38 (with conical profile, with self-centring function), designed to constitute a contrast element for the ring nut 16.

Screwed on the shank C of the pumping element of the ventricular-assistance device, the ring nut 16 is designed to ensure firm sealed connection of the duct 10 to the aforesaid pumping element.

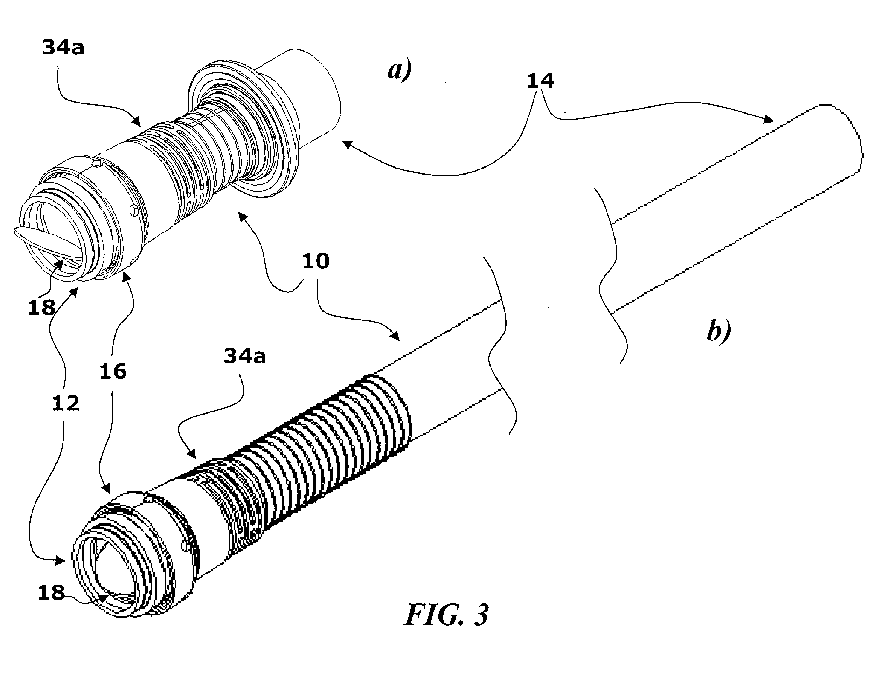

The sleeve body 34 is made of flexible material, for example polyethylene terephthalate (PTFE) or acetal resin (Delrin^{™}) and preferably has an appendage 34a (see Figure 3) with flexible structure, for example thanks to the construction according to a general slitted structure with Cardan-joint connections. The appendage 34a extends to cover at least partially the "reinforced" stretch of the duct 28 facing the valve 16 and is designed to function as "strain reliever" for the purpose of preventing build-up of undesired stresses between the sleeve body 34 and the duct 28.

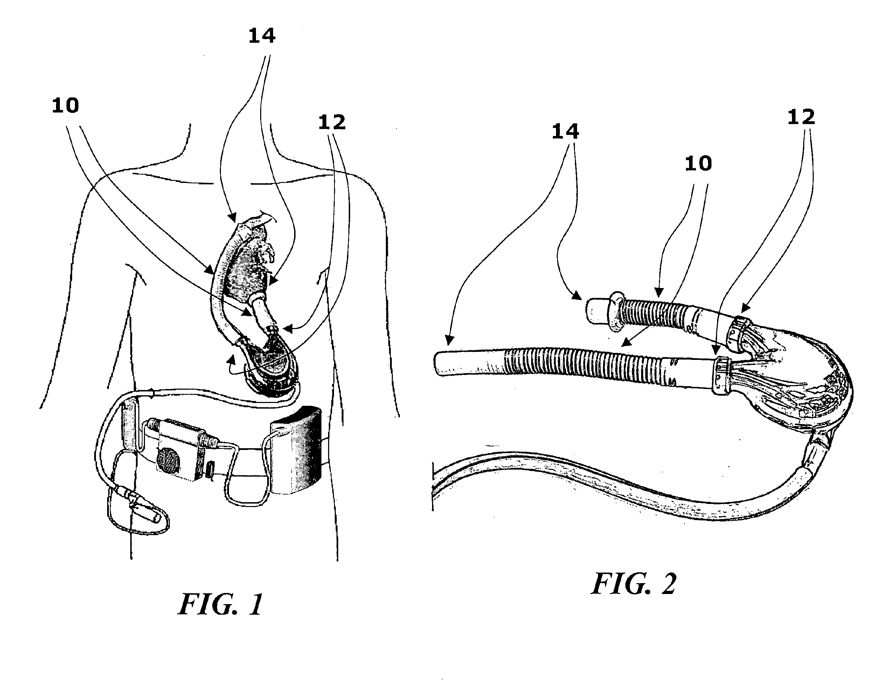

In this regard, the two parts a) and b) of Figure 3 are precisely designed to highlight how, according to the different operating needs that can be inferred from observation of Figures 1 and 2, the two ends 12 and 14 of the duct can either be at a certain distance from one another (with the body of the duct that is in itself able to absorb at least some stresses, such as those deriving from a different spatial orientation of the ends 12 and 14) or at a distance closer to one another, in conditions in which the aforesaid function of "strain reliever" becomes important.

In the solution described herein, the action of gripping of the ring nut 16 on the threaded shank C is not transmitted directly and in a rigid way to the end portion of the duct 10 itself, where the valve 18 is mounted.

When screwed on the shank C, the ring nut 16 is in fact forced against the shoulder 38 of the element 34. The corresponding action of thrust of the bodies 22 and 24 (with the valve 18 set between) against the shank C is exerted for the fact that the contrast surface 36 of the sleeve body 34 pushes the spacer ring 32, which (see the dashed representation of Figure 4) pushes the tubular element 31 that transmits the thrust to the body 26, which in turn pushes the body 24.

As regards the stresses applied on the end stretch 12 of the duct 10, where the valve 18 is located, the condition of rigid coupling between the ring nut 16 and the threaded shank C is hence, so to speak, "eased" by the elastically compliant character of the part of the sleeve body 34 that extends between the contrast surface 36 and the shoulder 38, as well as by the compressibility of the chain of elements 32-31-26 and 24.

In practice, the ensemble of the elements 22 and 24 that supports the valve 16 is "floating", in so far as it is isolated elastically, on the one hand, by the coating C1 set between the threaded shank C and the annular body 22 and, on the other, by the ensemble of the sleeve body 34 and the annular elements 26, 31 and 32. This floating-assembly arrangement is able to prevent the valve 18 from possibly being exposed to impact stresses that are too violent as a result of the energetic action of pumping performed by the pumping element of the ventricular-assistance device.

According to an important characteristic of the solution described herein, the entire internal surface of the duct 28 is provided with a coating 39 of haemocompatible material constituted typically by a haemocompatible polymer, such as a polyurethane.

This is preferentially a polyurethane substantially akin both to the polyurethane of the coating C1 of the shank C and to the polyurethane 40 that coats the elements 22 and 24 between which the valve 18 is mounted.

It follows that practically the entire line of flow of blood through the duct 10 illustrated herein is coated, upstream and downstream of the annular reinforcement 20 of the valve 18, with haemocompatible material.

The terms "upstream" and "downstream" with respect to the annular reinforcement 20 of the valve 18 is evidently intended to refer to the direction of flow of blood (which, as explained at the start of this description, is different, according to the function of the duct 10 and the orientation of the valve 18).

In other words, with reference to the example of embodiment illustrated herein, the end 12 and the tubular body 28 of the duct are provided with a internal coating 39, 40 of haemocompatible material that is continuous, with the sole exception of the annular reinforcement 20 of the valve 18.

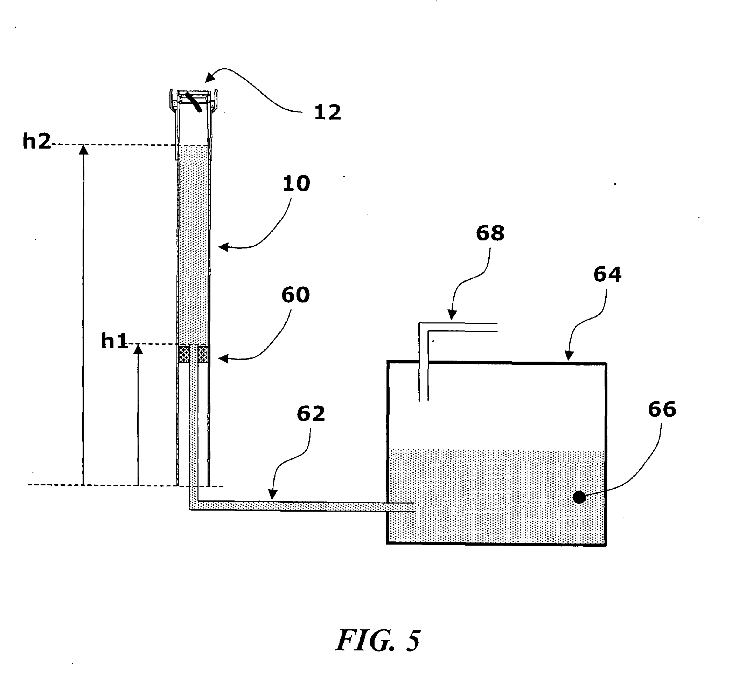

The coating with haemocompatible material of the internal surface of the ducts, which comes into contact with the blood, can be made, for example, using an apparatus like the one represented schematically in Figure 5 and following the process briefly illustrated in what follows.

The duct 10 is set in a vertical position with the end 12, proximal to the pumping element of the VAD, facing upwards. Introduced from beneath is a piston 60 having a diameter such as to enter the duct, thanks to its elasticity, but at the same time as to occlude its lumen. Said piston 60 is traversed, at the centre, by a tube 62, connected, at the other end, to a tank 64 containing an adequate volume of solution 66 of the haemocompatible material to be used for the coating, for example a solution of polyurethane in tetrahydrofuran. The tank 64 is hermetically sealed and is connected to a second duct 68, through which it is possible to pump air into it and so pressurize it, or sucking air out to obtain a negative pressure therein.

By appropriately positioning the piston 60 and pressurizing slowly and progressively the tank 64, the duct 10 is filled with the solution 66 in the desired stretch, for example between the levels h1 and h2 indicated in Figure 5. By then sucking the air out of the tank 64, the solution 66 is removed from the lumen of the duct 10, the surface of which remains, in the desired stretch, moistened by a film of solution. By removing the piston 60 from the lumen of the duct and ventilating, evaporation of the solvent and deposit of an adherent layer of the biocompatible material is obtained on the desired stretch of the internal surface of the duct. The thickness of said layer depends, not only upon the nature of the materials, but also upon the concentration of the solution used. By repeating the working cycle described above a number of times, appropriately changing h1, h2 each time, as well as possibly the concentration of the solution, it is possible to manage to coat the lumen of the duct integrally, with optimized profiles of thickness, to obtain a continuous uninterrupted surface of haemocompatible material.

In a particularly preferred way, the duct 10 is coated with a film of polyurethane with a thickness greater in the stretch of the duct 10 closer to the end for connection to the pumping element of the device, where it is necessary for the duct 10 to have a certain greater stiffness in regard to squeezing. The coating film made of haemocompatible material can be instead thinner in the areas more distant from the end connected to the pumping element of the ventricular-assistance device. Said characteristic facilitates the suture of the end of the duct to the artery, in the case of the outlet duct of the VAD, and enhances the qualities of flexibility, and hence of "compliance", of the duct 26.

It will be appreciated, on the other hand, that the solution described herein envisages the use of a duct with an end 12 that receives a valve and is coated internally with haemocompatible material.

In a specific way, with reference to the example of embodiment illustrated herein, the end 12 and the tubular body 28 of the duct are provided with an internal coating 39, 40 of haemocompatible material that is continuous, with the sole exception of the annular reinforcement 20 of the valve 18.

In a preferred way, there is envisaged the use of an outer sleeve (i.e., the element designated by 34 in the drawings) to obtain a chain for transmission of the stresses such as to reduce the stiffness of the anchorage of the valve. In the specific example of embodiment illustrated herein, the end 12 of the duct is constituted by: the ensemble of the elements 22 and 24 with the valve 18; the annular body 26 connected to the preceding ones by the sleeve 29; and, if present, by the spiral 30 anchored by means of the sleeve 31. The outer sleeve 34, with the ring 32, co-operates with the ring nut 16 for assembling the duct to the body of the pumping element.

Once again in a preferred way, the outer surface of the duct 10 is coated with an adherent film of biocompatible material.

Of course, without prejudice to the principle of the invention, the details of construction and the embodiments may vary, even significantly, with respect to what is described and illustrated herein, without thereby departing from the scope of the invention, as defined by the annexed claims.

## Claims

1. A duct (10) for carrying blood for ventricular-assistance devices, said duct (10) having a tubular body (28) and an end (12) having coupled thereto a valve (18) for enabling the flow of blood unidirectionally in the duct (10), in which:
- said valve (18) has an annular reinforcement (20) and said end (12) of the duct (10) is provided with a slot (22a, 24a) for receiving the annular reinforcement (20) of said valve (18); and
- said end (12) and said tubular body (28) of the duct are provided, upstream and downstream of the annular reinforcement (20) of the valve 18, with a continuous internal coating (39, 40) of haemocompatible material.

2. The duct according to Claim 1, in which said haemocompatible material is a haemocompatible polymer.

3. The duct according to Claim 1 or Claim 2, in which said haemocompatible material is a polyurethane.

4. The duct according to any one of the preceding claims, in which said end (12) of the duct (10) has walls that are radiused in the absence of discontinuity with the orifice of said annular reinforcement (20) of the valve.

5. The duct according to any one of the preceding claims, in which said end (12) of the duct comprises two annular bodies (22, 24) provided with mutually facing grooves (22a, 24a) that grip said reinforcement (20) of said valve (18) and said facing grooves (22a, 24a) defining said slot to one another.

6. The duct according to Claim 5, in which said two annular. bodies (22, 24) are made of a plastic material coated with said haemocompatible material.

7. The duct according to any one of the preceding claims, in which, in said tubular body (28) of the duct (10), said coating of haemocompatible material (38) has a thickness adjacent to said end (12) of the duct (10) greater than the remaining part of the tubular body (28) itself.

8. The duct according to any one of the preceding claims, in which said tubular body (28) of the duct (10) is provided, at least in an area adjacent to said end (12), with a stiffening reinforcement (30).

9. The duct according to Claim 8, in which said stiffening reinforcement (30) comprises a filiform element wound around said tubular body (28) of the duct (10).

10. The duct according to any one of the preceding claims, in which said tubular body (28) of the duct (10) carries associated thereto, in an area adjacent to said end (12), an annular element (26), which has a flared mouth part (26a) that surrounds said tubular body (28) of the duct (10).

11. The duct according to Claim 10, in which said tubular body (28) of the duct (10) is connected adhesively to said flared mouth part (26a).

12. The duct according to any one of the preceding claims, comprising:
- a ring nut (16) for connecting said duct (10) to a tubular shank (C);
- an elastically compliant sleeve body (34), which is fitted around said end (12) and which has a first contrast formation (36) and a second contrast formation (38) co-operating in a relationship of transmission of force, respectively, with said end (12) of the duct and said ring nut (16).

13. The duct according to Claim 12, in which said first contrast formation (36) and said second contrast formation (38) are located at opposite ends of said sleeve body (34).

14. The duct according to Claim 12 or Claim 13, in which said second contrast formation (38) is a conical shoulder of said sleeve body (34).

15. The duct according to any one of Claims 12 to 14, in which said first contrast formation (36) is an annular flange projecting within said sleeve body (34).

16. The duct according to any one of Claims 12 to 15, in which said first contrast formation (36) co-operates in a relationship of transmission of force with said end (12) through a spacer ring (32).

17. The duct according to Claims 10 and 16, in which said spacer ring (32) acts between said first contrast formation (36) and said annular element (26), which has a flared mouth part (26a) that surrounds said tubular body (28) of the duct (10).

18. The duct according to Claim 17, in which said annular element (26), which has a flared mouth part (26a) that surrounds said tubular body (28) of the duct (10), is completed by a further annular element (31) facing said spacer ring (32).

19. The duct according to any one of the preceding claims, the external surface of which is totally or partly coated with an adherent film of biocompatible material.
